# EUROPEAN PATENT APPLICATION

(11) **EP 2 759 282 A1**
(43) Date of publication of application: **30.07.2014**
(21) Application number: 12722744.5
(22) Date of filing: 23.03.2012
(51) Int. Cl.: A61F 5/453

(54) **URINE-COLLECTION DEVICE FOR MALE USE**

(30) Priority: 15.09.2011 ES 201130942
(71) Applicant: Garcia Calero, Diego Antonio, 03570 Villajoyosa (ES)
(72) Inventor: GARCÍA GÓMEZ, Alicia, E-03570 Villajoyosa (ES)
(74) Representative: Isern-Jara, Nuria
(86) International application number: PCT/ES2012/070192
(87) International publication number: WO 2013/038038

(57) **Abstract**

The device (1) comprises: a first elongate impermeable receptacle (2), for accommodating the patient's penis, with an entry opening (20) and an exit opening (21), for evacuation for the urine, connected to a drain line (3); and a second receptacle (4) with a closed end (40) and which is connected during operation to the first receptacle by means of a strip (41), and an open end (42) for accommodating the patient's scrotum. First affixing means (5) are provided for affixing the first receptacle, which include a first band (50) and a tying tape (51) coupled to the first band, which has two sections (510, 511) for tying to the patient's waist and the two tying portions of which are connected to the ends of the first band; and also second affixing means (6) for securing the second receptacle (4), which include a second band (60) and a tying tape (61) arranged in such a manner as to be coupled to said second band, which has two tying portions (610, 611) for securing to the patient's waist, and two portions of which are, respectively, connected to the ends of said second band.

## Description

### Scope and Prior Art

The invention generally relates to a urine-collection device for male use, for sanitary use in patients suffering from urinary incontinence, and also for use as a urine collector for bedridden patients.

Bedpan or bottle type urine-collection devices are currently known, but in addition to requiring the help of another person for use thereof these devices are a nuisance for the patient, making urination difficult, on many occasions due to the psychological aspects of a simple lack of privacy as a person is required to place and remove the container, and is very unpleasant for the sick and caregivers who have to place them, remove them, transport them and clean them after each use, and quite frequently during the night, with the ensuing inconveniences for them and other patients, who may be in the same ward in the case of hospitals.

From what is known by the inventor, there are currently no collector devices that can be secured to, attached to or hold the penis so as to receive urine, with the possibility of being attached to the body and directed to the reservoir, all without the need for a urethral probe, and thereby relieving the discomfort of leaks and discomfort due to the leaks wetting sheets, mattresses, undergarments, and outer garments in some cases.

### Object of the invention

Based on the previously described state of the art, the invention proposes the development of a urine-collection device as initially indicated in order to solve or at least alleviate the problems encountered and which can further be provided as a low-cost device and can therefore be discarded after use.

This is achieved by means of the characteristics indicated in claim 1. Other advantages are achieved by means of the characteristics indicated in the dependent claims.

The urine-collection device for male use according to the invention is characterised in that said device comprises:
- a first receptacle made from a substantially liquid-impermeable material in the form of a substantially elongated bag for inserting the patient's penis, having a proximal entry opening and a distal exit opening for evacuation of the urine, connected to a drain line, and
- a second receptacle, also in the form of a bag, having a closed end and which is connected during operation to the first receptacle, close to the proximal opening, by means of a strip, and an open end such that it can accommodate the patient's scrotum; and
- first affixing means being provided for securing the first receptacle which include a first band in the form of an open ring, inserted into the periphery of the proximal entry opening of said first receptacle and a tying tape coupled to the first band, which has two sections for securing to the patient's waist, and the tying portions of which are, respectively, connected during operation to the ends of said first band;
- and also second affixing means are provided for securing the second receptacle which include a second band in the form of an open ring, inserted into the periphery of the open end of said second receptacle and a tying tape arranged in such a manner as to be coupled to said second band, which has two tying portions also for securing to the patient's waist, the tying portions of which are, respectively, connected during operation to the ends of said second band.

According to a further characteristic of the invention, it is advantageous when the drain line is equipped with an outlet nozzle for coupling a closure member such as a sealing plug or a manual valve.

According to another characteristic of the invention it is advantageous that the first receptacle is provided as a plastic bag, preferably transparent, and that the second receptacle is formed from a woven or non-woven textile-type fabric, preferably for sanitary use.

Also according to a further characteristic of the invention, it is advantageous to have the first receptacle configured, at least in the area of the distal exit opening, in an inverted frustoconical arrangement, to facilitate the evacuation of the urine through the drain line.

According to yet another additional characteristic of the invention, it is advantageous when a piece of tube is inserted into the distal exit opening of the first receptacle to keep said opening permanently open, ensuring complete emptying of the first receptacle via the drain line.

### Brief description of the drawings

Other characteristics and advantages of the invention will be clearer from the description that follows, made with the aid of the accompanying drawings, illustrated by way of non-limiting example, in which:

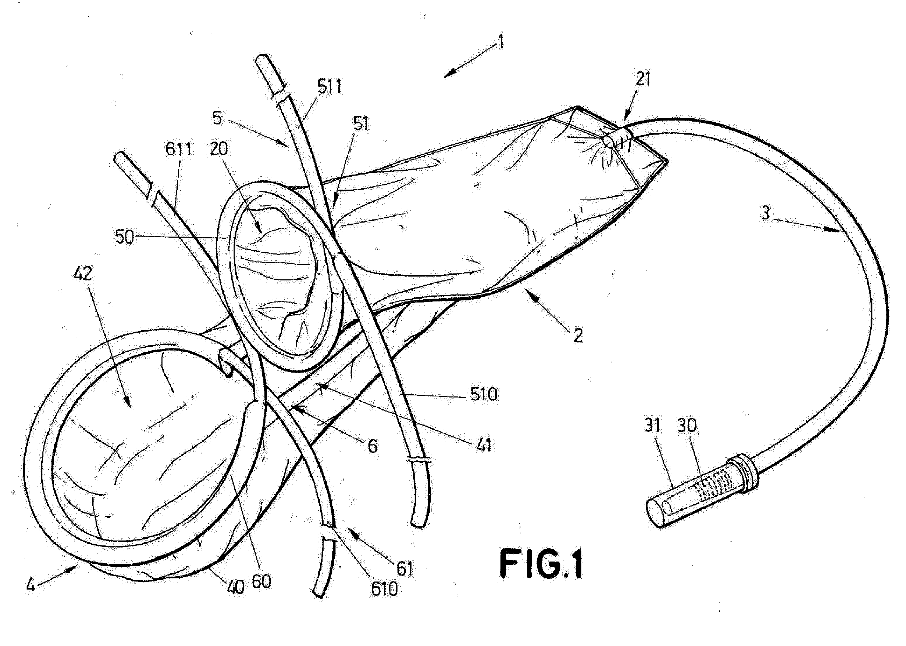

Figure 1 schematically shows a perspective view of a urine-collection device according to the invention.

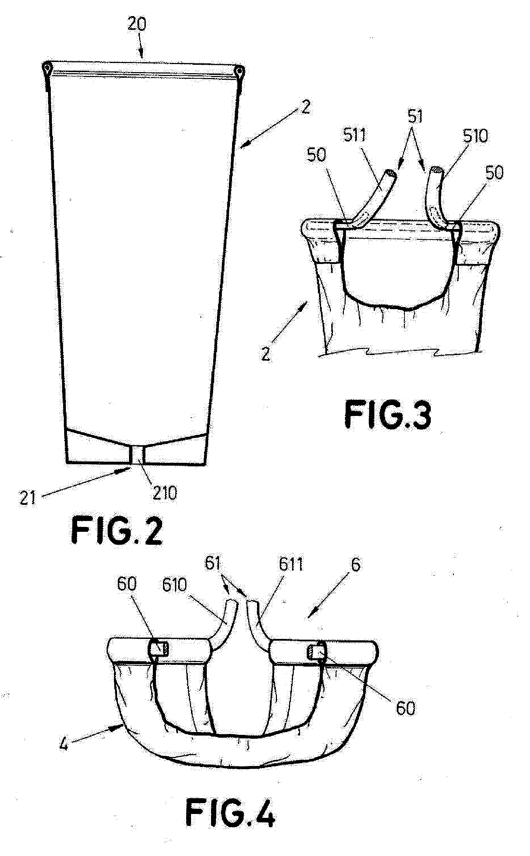

Figure 2 schematically illustrates the cross-section of the first urine-collection receptacle in a variation of an embodiment.
Figures 3 and 4 show respective partially sectioned views of the means for affixing the device to the patient's body.

### Detailed description of a preferred embodiment

As illustrated in the figures, the urine-collection device, generally indicated by reference 1, essentially comprises a first receptacle 2 in the form of an impermeable bag for collecting the urine, a second receptacle 4, both for securing to the patient as will be explained in detail below, and a drain tube 3 for evacuation of the urine.

It can be seen that the first receptacle 2, provided with a substantially liquid-impermeable material, has a substantially elongated shape for accommodating the patient's penis and has a proximal entry opening 20 and a distal exit opening 21 which is connected to a drain line 3 to evacuate the urine collected in said receptacle.

The second receptacle 4 which is also configured in the form of bag has a closed end 40 and an open end 42 such that it can accommodate the patient's scrotum. It can be seen that this second receptacle is connected during operation to the first receptacle 2 close to the proximal opening by means of a strip 41.

As shown in the figures, first affixing means 5 are provided for securing the first receptacle 2 which are comprised of a first band 50 in the form of an open ring which is inserted into the proximal entry opening 20 of said first receptacle and a tying tape 51 coupled to said first band, which has two sections 510, 511 for securing to the patient's waist, and the tying portions of which are, respectively, connected during operation to the ends of said first band;

It can further be seen that second affixing means 6 are provided for securing the second receptacle 4 which include a second band 60 in the form of an open ring, inserted into the periphery of the open end of said second receptacle and a tying tape 61 coupled to said second band, which has two tying portions 610, 611 for securing to the patient's waist, the tying portions of which are, respectively, connected during operation to the ends of said second band.

Thanks to the arrangement of the respective first and second bands 50, 60, in the form of an open ring, the ends of which are attached to the tying portions of the respective tying strips, they can be respectively adjusted to the patient's penis and scrotum and then secured around the patient's waist.

Advantageously, the drain line 3 has an outlet nozzle 30 at its free end for coupling a closure member 31, such as a sealing plug, and although not shown, this arrangement could be replaced by a manual closure valve. In this manner, the urine collected in the receptacle 2 can be evacuated to the outside or collected in a sanitary bag, which is not shown.

Also advantageously, the first receptacle 2 is provided as a plastic bag for sanitary use, preferably transparent; whereas for the purposes of patient comfort, the second receptacle 4 will be provided with material of the woven or non-woven fabric which is common for sanitary use.

In addition to facilitating the evacuation of the urine by gravity when the first receptacle with its distal opening 21 is directed downwards, it is advantageous to configure the first receptacle, at least in the area of said distal exit opening, in an inverted frustoconical arrangement.

It is also advantageous that a piece of tube (210) can be inserted into the distal exit opening (21) in order to achieve adequate evacuation of the urine that has been collected and to facilitate insertion of the drain tube.

As will be readily understood by those skilled in the art, what is described above is merely illustrative of a preferred embodiment of the invention such that technical modifications of any kind are possible, therefore the embodiments resulting from changes in form, dimensions and the like, as well as those resulting from application of the above development, must be considered within its scope, such that the invention shall be limited only by the scope of the following claims.

## Claims

1. Urine-collection device for male use **characterised by** the fact that said device (1) comprises:
- a first receptacle (2) made from a substantially liquid-impermeable material in the form of a substantially elongated bag for accommodating the patient's penis having a proximal entry opening (20) and a distal exit opening (21) for evacuation of urine connected to a drain line (3), and
- a second receptacle (4) also in the form of a bag having a closed end (40) and which is connected during operation to the first receptacle close to the proximal opening by means of a strip (41), and an open end (42) such that it can accommodate the patient's scrotum; and
- being provided with first affixing means (5) for securing the first receptacle which include a first band (50) in the form of an open ring, inserted into the periphery of the proximal entry opening (20) of said first receptacle and a tying tape (51) coupled to said first band, which has two sections (510, 511) for tying to the patient's waist, and the two tying portions of which are, respectively, connected during operation to the ends of said first band;
- and also provided with second affixing means (6) for securing the second receptacle (4) which include a second band (60) in the form of an open ring, inserted into the periphery of the open end of said second receptacle and a tying tape (61) arranged in such a manner as to be coupled to said second band, which has two tying portions (610, 611) for securing to the patient's waist, and two portions of which are, respectively, connected during operation to the ends of said second band.

2. Urine-collection device according to claim 1 **characterised by** the fact that the drain line (3) has an outlet nozzle (30) for coupling a closure member (31) such as a sealing plug on its free end.

3. Urine-collection device according to at least one of the preceding claims, **characterised by** the fact that the first receptacle (2) is a plastic bag, preferably transparent, the second receptacle (4) being provided with material of the woven or non-woven textile-type fabric.

4. Urine-collection device according to at least one of the preceding claims, **characterised by** the fact that the first receptacle is configured, at least in the area of the distal exit opening (21), in an inverted frustoconical arrangement to facilitate the evacuation of urine through the drain line (3).

5. Urine-collection device according to at least one of the preceding claims, **characterised by** the fact that a piece of tube (210) is inserted into the distal exit opening (21) of the first receptacle (2) to keep said opening permanently open, in order to completely empty the first receptacle (2).
